# EUROPEAN PATENT APPLICATION

(11) **EP 1 693 083 A1**
(43) Date of publication of application: **23.08.2006**
(21) Application number: 04805094.2
(22) Date of filing: 10.12.2004
(51) Int. Cl.: A61N 1/06

(54) **HIGH-FREQUENCY APPLICATOR FOR SKIN TISSUE THERAPIES**

(30) Priority: 10.12.2003 ES 200302835 U
(71) Applicant: INGENIERIA DEL LASER MADICO, S.L., E-08600 Berga (Barcelona) (ES)
(72) Inventor: LOPEZ LLURDA, José, E-08600 Berga (Barcelona) (ES)
(74) Representative: Thurgood, Alexander John
(86) International application number: PCT/ES2004/000551
(87) International publication number: WO 2005/056106

(57) **Abstract**

The invention relates to a high-frequency applicator for skin tissue therapies. The invention applicator comprises a vibrating element (5) with an active electrode (2) and two or more return electrodes (3) which are disposed parallel to the active electrode (2) on a shared conductive plate (4), said plate being separated from the active electrode (2), such as to form a bipolar applicator. Acccording to the invention, the plate (4) and the return electrodes (3) can be fixed or removable and changeable. Moreover, the plate is equipped with a rear locking element, such as one or more pins (7),for fixing same to the frame (1),and a contact for connecting the return conductor to the generator.

## Description

### Object of Invention

The present invention refers to a high frequency applicator for skin tissue therapies.

### Background of the Invention

In rehabilitation therapy and in skin treatments it has become usual to apply infrared radiation, cold or electromagnetical radiation of high or low frequency.

In the cases of application of high frequency electromagnetical radiation, high frequency generator normally has an applicator with only one electrode, and the return is realized through a second electrode placed near the first one, or through an applicator that includes two fixed plates situated in the same plane; in this case, one of the plates is used as an active electrode and the other one as a return electrode; the depth of the action is constant and not combined with other therapeutic effect.

### Description of the Invention.

The high frequency applicator for skin tissue therapy being the object of this invention allows for more correct and efficient application of electromagnetical fields of greater or lesser depth in a simple way, in combination with a vibrating movement.

For its use, the applicator comprises a casing with an active vibrating electrode mounted inside of it. This electrode, of a generic cylindrical form, has around it two or more return electrodes, parallel and separated between them. These return electrodes are situated on a fixing dismountable plate with electrical conductive properties.

The external surface of active electrode, made preferably of ceramic plasma, is covered with polyamide and/or Teflon, which, apart from providing necessary insulation, makes this external surface smooth.

The part of the electrode situated inside of the casing has a vibrating element, such as en electric motor with off-centre axis, so that the vibration is transmitted to the said active electrode. To make this vibration possible, the electrode has around it two elastic o-rings that connect it to the casing making the movement possible and providing insulation between the two elements.

As it has been mentioned, return electrodes are fixed on a junction plate that has electrical conductive properties; its preferable form is that of an elliptic crown, situated around the active electrode at a certain distance.

The said electrodes are preferably made of stainless steel or similar material, covered with an insulating material and situated in line with the ends of one or both axes, major and minor of the elliptical plate. In this way, the applicator provides electromagnetical fields of different range and penetration due to separation between return electrodes and active electrode, making it possible to apply more or less penetrating therapies depending on the position, in which the applicator is situated on patient's skin.

It has been foreseen that the plate that bears the return electrodes can be easily extracted and replaced. For this purpose, the said plate has on its rear part one or several anchorages or locking pins, apart from an electrical contact for return connection.

On its front part, on the plate that bears return electrodes, the applicator has light indicators of operation. These indicators indicate if the applicator is active and the frequency that is being used.
This applicator has several advantages due to the fact that the electrode is bipolar and it is possible to achieve greater or lesser depth of penetration of energy field with the change of electrodes from major to minor axis of the ellipse. Besides, the vibrating movement of active electrode contributes to therapeutic effect.

### Description of the Figures.

To complement the description that is being done and to facilitate the understanding of the characteristics of the invention, this descriptive brief is accompanied with a set of figures, which, having illustrative and not limitative nature, show the following:

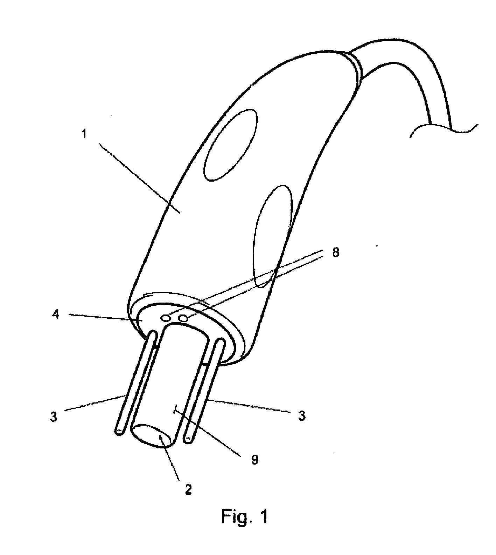

- Figure 1 shows a prospective view of the applicator with two return electrodes

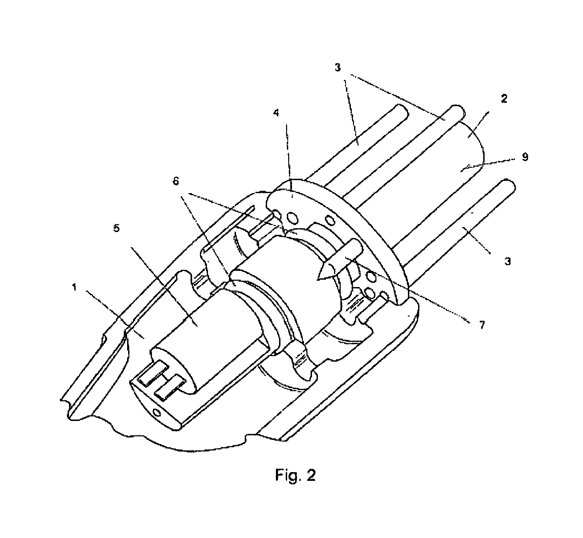

- Figure 2 shows a partial prospective view of the applicator front area with four return electrodes; a part of the casing has been removed in order to allow for observation of the vibrating element connected to the active electrode and the o-rings for assembly of the electrode in the casing.

### Preferred Embodiment of the Invention

The figures, according to the numeration adopted, show a preferred, but not limitative, embodiment of the applicator that comprises a casing (1) and, on its front part, an active electrode (2) of a cylindrical form, which emerges from the central orifice of a plain plate (4), on an elliptical form, without touching it. In other embodiments, the plate (4) could have round, square or any other form.

The said active electrode (2) has on its rear part situated inside the casing (1) a vibrating element (5) such as a motor with off-centre axis, and several elastic o-rings (6) inserted on the supports of the active electrode (2) with the casing, allowing for vibration of the electrode. The active electrode (2) made of ceramic plasma has on its exterior surface (9) an insulating covering of polyamide and/or Teflon.

Two o more return electrodes (3) are fixed on a bearing plate (4) in parallel to the active electrode (2) and in line with major and minor axes of the elliptical plate (4).

Return electrodes (3) are made of stainless steel and covered with an insulating material. The plate (4) has a least one locking element or a rear pin (7) for coupling of the assembly plate - return electrodes (4-3) over the casing (1) of the applicator. The plate (4) also has a contact (not shown) for connection of the return conductor to a high frequency generator.

On its front part, the applicator has one or two optical indicators (8), for example, light diodes (LED) that indicate the operative state of the applicator and the applied frequency.

Having sufficiently described the nature of the invention, as well as an example of its preferred embodiment, we hereby state for appropriate purposes that the materials, form, dimensions and position of the elements described above can be modified, on condition that this modification will not lead to an alteration of the essential characteristics of the invention, which are claimed below.

## Claims

1. High frequency applicator for skin tissue therapies of the type that is connected to a high frequency generator, **characterized by** the fact that it comprises a vibrating element (5) on the active electrode (2), and by the fact that is comprises at least two or more return electrodes (3), parallel to the active electrode (2) and situated on a common conductive plate (4), separated from the said active electrode (2), forming a bipolar applicator.

2. Applicator according to claim 1, **characterized by** the fact that the plate (4) and the return electrodes (3) can be fixed or removable and changeable.

3. Applicator according to clam 2, **characterized by** the fact that the plate (4) comprises a rear locking element for its fixation on the casing (1), such as one or several pins (7), and by the fact that it comprises a contact for connection of the return conductor to the generator.

4. Applicator according to claim 1, **characterized by** the fact that the plate (4) has the form of an elliptical crown, with return electrodes (3) positioned over major and/or minor axes of the said ellipse, suitable for application of an energy field of greater or lesser penetration depth on the tissues.

5. Applicator according to claim 1, **characterized by** the fact that the vibrating element (5) is a motor with off-centre axis.

6. Applicator according to claim 1, **characterized by** the fact that the return electrodes (3) are made of stainless steel and their surface is covered with a layer of an insulating material.

7. Applicator according to claim 1, **characterized by** the fact that the casing (1) comprises several light indicators (8) that indicate the operative state of the applicator and of the frequency applied.
